(19) Europäisches Patentamt
European Patent Office
Office européen des brevets

(11) EP 1 393 708 B1

(12) EUROPEAN PATENT SPECIFICATION

(45) Date of publication and mention of the grant of the patent:
**02.07.2014 Bulletin 2014/27**

(21) Application number: **02730869.1**

(22) Date of filing: **03.06.2002**

(51) Int Cl.:
***A61K 8/46*** ***(2006.01)*** ***A61Q 5/04*** ***(2006.01)***

(86) International application number:
**PCT/JP2002/005419**

(87) International publication number:
**WO 2002/098381 (12.12.2002 Gazette 2002/50)**

# (54) METHOD OF TREATING HAIR

VERFAHREN ZUR BEHANDLUNG VON HAAREN

PROCEDE DE TRAITEMENT DES CHEVEUX

(84) Designated Contracting States:
**DE ES FR GB IT**

(30) Priority: **04.06.2001 JP 2001168078**

(43) Date of publication of application:
**03.03.2004 Bulletin 2004/10**

(73) Proprietor: **KAO CORPORATION**
**Tokyo 103-8210 (JP)**

(72) Inventors:
- **TSUCHIYA, Masaru,**
**c/o KAO CORP. Research Lab.**
**Sumida-ku, Tokyo 131-8501 (JP)**
- **EZURE, Mikako,**
**c/o KAO CORP. Research Lab.**
**Sumida-ku, Tokyo 131-8501 (JP)**
- **ITO, Takashi,**
**c/o KAO CORP. Research Lab.**
**Sumida-ku, Tokyo 131-8501 (JP)**

(74) Representative: **HOFFMANN EITLE**
**Patent- und Rechtsanwälte**
**Arabellastrasse 4**
**81925 München (DE)**

(56) References cited:

| | |
|---|---|
| **EP-A- 0 529 437** | **EP-A- 0 629 395** |
| **EP-A1- 0 529 437** | **EP-A1- 0 629 395** |
| **DE-A- 4 411 856** | **DE-A- 19 536 423** |
| **GB-A- 690 866** | **JP-A- 6 293 618** |
| **JP-A- 7 101 840** | **JP-A- 8 092 043** |
| **JP-A- 58 222 008** | **US-A- 3 482 581** |

- **DATABASE WPI Week 199524 Thomson Scientific, London, GB; AN 1995-182875 XP002559187 & JP 07 101840 A (KAO CORP) 18 April 1995 (1995-04-18)**

Remarks:
The file contains technical information submitted after the application was filed and not included in this specification

EP 1 393 708 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

This invention relates to a extremely curly hair correction process involving reduction of hair keratin and a process for maintaining or restoring the thus-corrected state.

### Background Art

To straighten naturally curly hair or extremely curly hair, several extremely curly hair correction processes are practiced at beauty salons and the like. These processes include a process called "straight perm". This process comprises applying a first-pack straight perm composition, which contains a hair keratin reducing substance, to curly hair to fix the hair in straight configuration, allowing the hair to stand for a predetermined time, washing off the first-pack straight perm composition with water, and then treating the hair with a second-pack composition which contains an oxidizing agent. This process has a merit that it is relatively simple and convenient, but involves demerits that the correcting effect is weak and the treated hair tends to prematurely return with days into the form of the curly hair.

There is also a process called "ironing extremely curly hair correction". According to this process, a first-pack composition with a hair keratin reducing substance contained therein is applied to curly hair, the hair is left over for a predetermined time, the first-pack composition is rinsed off with water, the hair is shaped into straight configuration by a high-temperature iron controlled around 160°C, and then, the hair is treated with a second-pack composition which contains an oxidizing agent. This process has a high correcting effect and persistency compared with the above-mentioned straight perm, but requires labor and time. This process is also accompanied by a problem in that natural waves tend to remain at less voluminous hair tip portions.

Whichever process is used, however, a return into the form of the curly hair takes place through repetition of shampooing or the like after the correction treatment of the extremely curly hair. To correct the extremely curly hair again, straight perm or ironing extremely curly hair correction has to be applied again. Repetition of such treatment leads to a problem that the hair is seriously damaged.

Not limited to hair straightening but also even when various wavy perms are applied to curly hair or extremely curly hair, an insufficient extremely curly hair correcting effect results in a problem that the natural curliness or curliness of the hair remains without complete correction, the resulting waves are not styled neat and hence, the finish is not attractive.

As a process capable of solving the above-mentioned problems, a technique has been proposed to straighten hair by using a particular organic salt or a salt thereof, which has a curly hair correcting effect under an acidic condition, in combination with an organic solvent having a hair penetration promoting effect (JP-A-08092043). This process is, however, not fully comparable in extremely curly hair correcting effect with the conventional straight perms. Another extremely curly hair correcting process has also been proposed, which comprises applying a composition, which contains a compound having a pKa of 5 or smaller and at least one carboxylic acid group or sulfonic acid group and has a pH of from 2 to 5, and then conducing an oxidizing treatment (JP-A-07101840). This process is, however, not sufficient in extremely curly hair correcting effect, either.

### Disclosure of the Invention

An object of the present invention is to solve these conventional problems and to provide a hair treatment process having a very high extremely curly hair correcting effect and very high persistency of the effect. Another object is to provide a process for easily achieving maintenance and restoration of the extremely curly hair correcting effect, which has been imparted by practicing the hair treatment process, without giving a damage to the hair.

The present inventors have found that a extremely curly hair correcting effect and persistency of the effect, which are higher than those available from the conventional straight perms, can be obtained when, in a state that the hair structure is relaxed by reductive cleavage of disulfide bonds in the hair in the course of a so-called permanent treatment making use of a first-pack composition with a hair keratin reducing substance contained therein and a second-pack composition with an oxidizing agent contained therein, an organic acid having a specific structure is applied and the pH is then controlled to 1 to 5 with a buffering agent to have the organic acid absorbed in a large amount in hair. The present inventors have also found that this process makes it possible to obtain a high extremely curly hair correcting effect even only by a simple shaping action such as combing and further that use of a heating device such as a high-temperature iron can obtain a still higher extremely curly hair correcting effect. Further, the present inventors have also found that, when hair is wound on rods as a styling action or otherwise styled to impart waves to the hair, neatly styled curling is feasible even if the hair has significant natural curliness or curliness, because the hair is styled into curls while the natural curliness or curliness is being corrected.

The present inventors have also found that the extremely curly hair correcting effect can be easily maintained or restored without giving a damage to the hair by causing an aqueous treatment composition - which contains an organic acid having a specific structure or a salt thereof, has buffer capacity and has a pH of from 1 to 5 - to act on the hair previously subjected to extremely curly hair correction by the above-described method.

The present invention provides a hair treatment process comprising the following steps (2-1) to (2-5) in at least one of which shaping of hair is conducted with optional use of a heating device as needed, and is conducted with a first pack comprising the organic acid (A) as defined below:

Steps:

(2-1)- applying the first pack, which comprises a hair keratin reducing substance, to the hair, and after allowing the hair to stand for 1 to 120 minutes,
(2-2) applying an intermediate treatment, which has buffer capacity and has a pH of from 1 to 5, to the hair,
(2-3) rinsing the hair with water as needed,
(2-4) applying a second pack, which comprises an oxidizing agent, to the hair, and after allowing the hair to stand for 1 to 60 minutes,
(2-5) rinsing the hair with water, and drying the hair.

The organic acid (A) is a compound selected from octanesulfonic acid and ethylhexanesulfonic acid or a sodium salt thereof, a potassium salt thereof or an ammonium salt thereof.

The present invention also provides a hair treatment process which comprises applying an after-treatment - which comprises the organic acid (A), has buffer capacity and has a pH of from 1 to 5 - to the hair treated by the above-described processe, allowing the hair to stand for 1 to 120 minutes, rinsing the hair with water, and then drying the hair.

According to the hair treatment process according to the present invention, an extremely high, extremely curly hair correcting effect can be obtained by a procedure as simple as conventional straight perm. Further, subsequent to the practice of the extremely curly hair correcting process, the corrected state can be maintained or, after returned into the extremely curly state, the corrected state of the hair can be restored, both by a routine aftercare at home without damaging the hair. It has, therefore, become possible to avoid a damage to the hair by a treatment such as a further straight perm or additional ironing which has heretofore been required to maintain a straightened state.

## Best Modes for Carrying Out the Invention

In the above-described hair treatment process comprising the steps (2-1) to (2-5) it is necessary for the first pack to contain the organic acid (A). It is, however, preferred that the second pack also contains the organic acid (A).

When these first pack, intermediate treatment and second pack contain the organic acid (A), its content in each treatment may range preferably from 0.1 to 40 wt.%, more preferably from 1 to 30 wt.%, notably from 2 to 20 wt.%.

The expression "to have buffer capacity" as used herein means that a value B determined in accordance with the following formula by using as a scale the concentration of a base required to raise the pH of a 10 wt.% aqueous solution of a composition by 1 from the initial value at 25°C (hereinafter called "B value") is 0.001 gram equivalent/L or greater.

$$B = |dC_B/dpH|$$

where $C_B$ indicates the ionic concentration (gram equivalent/L) of the base.

Measuring method: An aliquot (10 g) of the composition is weighed, to which water is added to 100 mL. The pH at this time is measured. To the solution, a 1 N aqueous solution of sodium hydroxide is then added to determine the volume (**X** mL) of the 1 N aqueous solution of sodium hydroxide required to raise the pH by 1. The value B is then calculated by B = **X** x 10/1000 gram equivalent/L.

To allow the aqueous composition to have buffer capacity at pH 1 to 5, it is necessary for the aqueous composition to contain as a buffering agent an organic or inorganic acid the acid dissociation constant pKa at 25°C of which (at least one pKa when the acid has plural acid dissociation constants) ranges from 1 to 5, and preferably also to contain its salt. Examples of the acid can include carboxylic acids and phosphoric acids, such as citric acid, lactic acid, malic acid, tartaric acid, glycolic acid, succinic acid, benzoic acid, and pyrrolidonecarboxylic acid. Illustrative of their salts are their alkali metal salts such as their sodium salts and potassium salts, their alkaline earth metal salts; and their ammonium salts. The possession of buffer capacity by the aqueous composition can enhance the extremely curly hair correcting effect. This can be considered to be attributed to the possibility of holding the pH at a low value by the possession of buffer

capacity, since it is advantageous to control the pH at a lower value upon causing the anionized organic acid (A) to be adsorbed and immobilized like ionic bonding at cation sites formed as a result of cutting of ionic bonds in hair under an acidic condition.

The first pack for use in the present invention contains the hair keratin reducing substance. Examples of the hair keratin reducing substance can include thioglycolic acid, thiolactic acid, thioglycerol and glyceryl monothioglycolate, their salts such as their ammonium and sodium salts, and their derivatives; and cysteine, cysteine hydrochloride, N-acetylcysteine, sulfites and hydrogensulfites. Two or more of these hair keratin reducing substances can be used in combination. Its content may range preferably from 0.1 to 20 wt.%, more preferably from 1 to 10 wt.%, notably from 2 to 8 wt.% in the first pack.

To adjust the reducing power, it is also possible to add dithiodiglycolic acid or dithiodilactic acid, which is the dithio compound of the corresponding hair keratin reducing thiol compound described above, a salt thereof or the like.

The pH of the first pack may be adjusted preferably to 5 to 11, more preferably 6 to 10, notably 7 to 9.6.

The intermediate treatment for use in the present invention has buffer capacity, specifically a B value of 0.001 gram equivalent/L or greater, preferably 0.01 gram equivalent/L or greater, notably 0.01 to 0.2 gram equivalent/L. The intermediate treatment can preferably contain a buffering agent in a proportion of from 1 to 20 wt.%. Its pH ranges from 1 to 5, preferably from 1 to 4, more preferably from 1 to 3.5, notably from 2.5 to 3.5.

To be able to confirm that the pH has fallen within the range of from 1 to 5 upon mixing the intermediate treatment with the first pack on the hair, the intermediate treatment may contain a pH indicator, for example, methyl yellow, methyl orange, methyl red or bromophenol blue.

As a special form of the intermediate treatment, it may be contemplated, for example, to mix a suitable amount of a solid acid (citric acid or the like) with the first pack during application of the first pack to the hair such that the pH is lowered to 1 to 5; or to coat a solid acid with a sustained-dissolution coating agent and to mix a suitable amount of the thus-coated solid acid with the first pack shortly before or during application of the first pack to the hair such that the pH is lowered to pH 1 to 5 at a stage that a certain time has elapsed. Usable examples of the coating agent can include watersoluble high molecular compounds such as polyethylene glycol, gypsum films, hydrophobic substances such as calcium fluoride, solid fat preparations, and surfactants.

The second pack for use in the present invention contain the oxidizing agent. Examples of the oxidizing agent can include sodium bromate, sodium perborate and hydrogen peroxide, with hydrogen peroxide being particularly preferred. Two or more of these oxidizing agents can be used in combination. Its content may preferably range from 0.1 to 20 wt.% in the second pack.

In the hair treatment process comprising the steps (2-1) to (2-5), the intermediate treatment has buffer capacity and its pH ranges from 1 to 5. It is, therefore, not essential for the second pack to be one having buffer capacity and pH 1 to 5, although it is preferred to have buffer capacity and pH 1 to 5. When this second pack has buffer capacity, it is preferred to contain a buffering agent in a proportion of from 1 to 20 wt.%.

These first pack, intermediate treatment and second pack may preferably contain an organic solvent to facilitate penetration of the organic acid (A) or salt thereof into the hair. Preferred examples of the organic solvent can include those represented by any one of the following formulas (i), (ii), (iii), (iv) and (v).

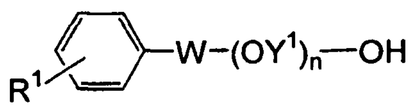 (i)

wherein $R^1$ represents a hydrogen atom, a linear or branched alkyl group having 1 to 4 carbon atoms or a linear or branched alkoxy group having 1 to 4 carbon atoms, W represents a single bond, a methylene group or a linear or branched alkylene group having 2 to 4 carbon atoms, $Y^1$ represents a linear or branched alkylene group having 2 to 4 carbon atoms, and n denotes an integer of from 0 to 3.

$$R^2\text{-}(OY^2)_m\ OH \qquad \text{(ii)}$$

wherein $R^2$ represents a linear, branched or cyclic alkyl group having 4 to 7 carbon atoms, $Y^2$ represents a linear or branched alkylene group having 2 to 4 carbon atoms, and m denotes an integer of from 0 to 3.

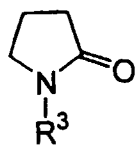 (iii)

wherein $R^3$ represents a linear or branched alkyl group having 1 to 4 carbon atoms.

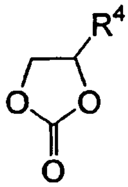

(iv)

wherein $R^4$ represents a hydrogen atom or a linear or branched alkyl group having 1 to 4 carbon atoms.

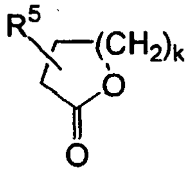

(v)

wherein $R^5$ represents a hydrogen atom or a linear or branched alkyl group having 1 to 4 carbon atoms, and k denotes an integer of 1 or 2.

In the formulas (i) to (v), examples of the linear or branched alkyl groups having 1 to 4 carbon atoms and represented by $R^1$, $R^3$, $R^4$ and $R^5$ can include methyl group, ethyl group, propyl group, isopropyl group, butyl group, isobutyl group, sec-butyl group and tert-butyl group. Examples of the linear or branched alkoxy group having 1 to 4 carbon atoms and represented by $R^1$ can include methoxy group, ethoxy group, propoxy group, isopropoxy group and butoxy group. Examples of the linear, branched or cyclic alkyl group having 4 to 7 carbon atoms and represented by $R^2$ can include butyl group, pentyl group, hexyl group, heptyl group, isobutyl group, cyclopentyl group and cyclohexyl group. Further, examples of the linear or branched alkylene groups having 2-4 carbon atoms and represented by X, $Y^1$ and $Y^2$ can include can include ethylene group, trimethylene group, propylene group and tetramethylene group.

Illustrative organic solvents (i) are 2-phenoxyethanol, benzyl alcohol, 2-phenylethanol and 2-benzyloxyethanol; illustrative organic solvents (ii) are n-butanol, ethylene glycol mono(n-butyl) ether and diethylene glycol mono(n-butyl) ether; illustrative organic solvents (iii) are N-methylpyrrolidone and N-ethylpyrrolidone; illustrative organic solvents (iv) are ethylene carbonate and propylene carbonate; and illustrative organic solvents (v) are $\gamma$-butyrolactone, $\gamma$-valerolactone and $\gamma$-caprolactone. Among these, the organic solvents (i), especially benzyl alcohol and 2-benzyloxyethanol are preferred.

It is also preferred to add a lower alkanol such as ethanol or isopropanol, a polyol such as propylene glycol, hexylene glycol or glycerin, or a mono-lower alkyl ether of ethylene glycol or diethylene glycol.

Two or more organic solvents may be used in combination. Its content may range preferably from 1 to 50 wt.%, more preferably from 3 to 45 wt.%, notably from 5 to 40 wt.% in each pack and treatment.

In the step (2-1), the amount of the first pack to be applied may range preferably from 0.1 to 4 times by weight, notably from 0.5 to 2.5 times by weight as much as the hair to be treated. Depending upon the degree of natural curliness, curliness or the like, the thickness and the damage condition of the hair to be treated, the time of treatment with the first pack may be suitably adjusted within a range of from 1 to 120 minutes. During the treatment, the hair may be heated if necessary. It is preferred to control the temperature at 30 to 55°C, especially at 35 to 50°C around the hair.

In the step (2-2), the intermediate treatment is applied to the hair which has been treated with the first pack. The amount of the intermediate treatment to be applied is suitably adjusted depending upon the pH of the first pack, the pH and buffer capacity of the intermediate treatment, and the like. Specifically, the intermediate treatment may be applied in an amount such that subsequent to its mixing with the first pack as an aqueous alkaline solution, the pH may fall within a range of from 1 to 5, preferably from 2 to 4, more preferably from 2.5 to 3.8. The amount of the intermediate treatment to be applied may be preferably 1 to 50 times by weight, notably 2 to 10 times by weight as much as the amount of the first pack applied. Depending upon the degree of natural curliness, curliness or the like, the thickness and the damage condition of the hair to be treated, the time of treatment with the intermediate treatment may be suitably adjusted within a range of from 0.1 to 60 minutes. During the treatment, the hair may be heated if necessary. It is preferred to control the temperature at 30 to 55°C, especially at 35 to 50°C around the hair. Incidentally, the correcting effect can be enhanced still further by conducting additional treatment with the intermediate treatment, which contains the organic acid (A), after the hair is rinsed with water subsequent to its successive treatment with the first pack, which contains the organic acid (A), and the intermediate treatment.

It is preferred to conduct the rinsing of the step (2-3) so that the still remaining treatment with the hair keratin

reducing substance contained therein can be rinsed off.

In the step (2-4), the amount of the second pack to be applied may range preferably from 0.1 to 3 times by weight, notably from 0.5 to 2.5 times by weight as much as the hair to be treated. Depending upon the hair keratin reducing power of the first pack, the time of treatment with the first pack, the oxidizing power of the second pack, and the like, the time of treatment with the second pack may be suitably adjusted within a range of from 1 to 60 minutes.

In the step (2-5), the aqueous treatment on the hair is first rinsed off, and after shampooing and rinsing as needed, the hair is dried.

The shaping is conducted in any one, two or more of the above-described steps. This shaping itself can be conducted in a similar manner as in conventional permanent treatments (straight perm, wavy perm, ironing perm). In the case of straightening, a sufficient extremely curly hair correcting effect can be obtained even by a simple procedure that the hair is smoothed into straight configuration by a brush or the like while the first pack, the second pack or the intermediate treatment still remains on the hair. When an iron is used, a high extremely curly hair correcting effect can also be obtained by ironing the hair after the aqueous treatment is rinsed off in the step (2-3) or (2-5).

When an iron is used for shaping, the temperature of the iron may be set preferably at 70 to 180°C, notably at 140 to 180°C. Upon ironing, a treatment with a hair protecting ingredient or the like contained therein can be used as needed. The use of the iron makes it possible to obtain a still higher extremely curly hair correcting effect.

The hair treated by the above-described process is also excellent in the persistency of the extremely curly hair correcting effect. When general hair care (shampooing, rinsing and the like) is repeated routinely, a clear difference is observed in persistency between the hair treated by the above-described process and the hairs treated by conventional straight perm.

When an after-treatment which contains the organic acid (A) or salt thereof and has buffer capacity and pH 1 to 5 is applied to the hair upon elapsed time of several days to several weeks subsequent to its treatment by the above-mentioned process and, after the hair is left over for 1 to 120 minutes, the hair is rinsed with water and is then dried, the extremely curly hair correcting effect can be maintained for a longer period or can be restored even after an elapse of a longer period.

The after-treatment employed here has buffer capacity, and its B value is 0.001 gram equivalent/L or greater, preferably 0.01 gram equivalent/L or greater, notably 0.01 to 0.2 gram equivalent/L. The after-treatment may contain a buffering agent in a proportion of from 1 to 20 wt.%. Its pH may range from 1 to 5, preferably from 1 to 4, more preferably from 1 to 3.5, notably from 2.5 to 3.5.

The content of the organic acid (A) or salt thereof in the after-treatment may range preferably from 0.1 to 40 wt.%, more preferably from 1 to 30 wt.%, notably from 2 to 20 wt.%. To facilitate the penetration of the organic acid (A) or salt thereof into the hair, the after-treatment may preferably contain an organic solvent. Preferred examples of the organic solvent can be those represented by any one of the formulas (i), (ii), (iii), (iv) and (iv) described above. Two or more organic solvents may be used in combination. Its content may range preferably from 1 to 50 wt.%, more preferably from 3 to 45 wt.%, notably from 5 to 40 wt.% in the whole composition.

As the after-treatment is no longer required to contain the hair keratin reducing substance or the oxidizing agent, the above-described treatment does not cause a hair damage which would otherwise occur by such a substance or agent. Further, the after-treatment can be used very conveniently as it can be formulated into a shampoo, a rinse, a pre-shampooing treatment or the like. The after-treatment can be applied no matter whether the hair is dry or wet. The amount of the after-treatment to be applied may range preferably from 0.1 to 3 times by weight, notably from 0.5 to 2.5 times by weight as much as the hair to be treated, although it should be suitably adjusted depending on its preparation form and the like. The time of treatment with the after-treatment may be suitably adjusted within a range of from 1 to 120 minutes. During the treatment, the hair may be heated if necessary. It is preferred to control the temperature at 30 to 55°C, especially at 35 to 50°C around the hair.

Preferably, the first pack, second pack, intermediate treatment and after-treatment in the present invention may each contain water as a medium and be in the form of a liquid or in the form of a gel with a viscosity increasing agent contained therein. In addition to the above-mentioned ingredients, they can contain a variety of perfume and/or cosmetic ingredients as needed. Illustrative of such perfume and/or cosmetic ingredients are pH adjusters (ammonia, monoethanolamine, 2-propanolamine, sodium hydroxide, citric acid, sodium citrate, lactic acid, sodium lactate, phosphoric acid, etc.), viscosity increasing agents (hydroxyethylcellulose, xanthan gum, polyacrylic acid derivatives, etc.), anionic surfactants, cationic surfactants, amphoteric surfactants, nonionic surfactants, semi-polar surfactants, cosmetic oils, silicones, anionic polymers, cationic polymers, amphoteric polymers, fragrances, colorants, preservatives, ultraviolet absorbers, hair protecting ingredients, touch feel improvers, humectants, chelating agents, hair growth promotion ingredients, and so on.

**Examples**

Example 1 and Comparative Example 1 (not according to the invention as claimed)

The following first pack, intermediate treatment and second pack were prepared, and were employed for the below-described extremely curly hair correction treatments and their ranking.

First Pack X [sodium 2-naphthalenesulfonate (2-NSA added)]

| | (wt.%) |
|---|---|
| Monoethanolamine thioglycolate (50 wt.%) | 14.0 |
| Monoethanolamine | q.s.(to pH 9.0) |
| Sodium 2-naphthalenesulfonate | 6.0 |
| 2-Benzyloxyethanol | 7.5 |
| Ethanol | 10.0 |
| Disodium edetate | 0.5 |
| Hydroxypropyl xanthan gum | 2.0 |
| Purified water | Balance |
| | 100.0 |

First Pack Y [2-NSA free]

2-NSA was not added in the composition of the first pack X, and the composition was balanced with purified water.

Intermediate Treatment X [2-NSA added]

| | (wt.%) |
|---|---|
| Lactic acid | 5.0 |
| Citric acid | 5.0 |
| Sodium 2-naphthalenesulfonate | 6.0 |
| 2-Benzyloxyethanol | 7.5 |
| Ethanol | 10.0 |
| 48 wt.% aq. soln. of caustic soda | q.s.(to pH 3.0) |
| Purified water | Balance |
| | 100.0 |

Intermediate Treatment Y [2-NSA free]

| | (wt.%) |
|---|---|
| Lactic acid | 5.0 |
| Citric acid | 5.0 |
| 48 wt.% aq. soln. of caustic soda | q.s.(to pH 3.0) |
| Purified water | Balance |
| | 100.0 |

Second Pack X [2-NSA added]

| | (wt.%) |
|---|---|
| 35 wt.% hydrogen peroxide | 5.71 |
| Lactic acid | 5.0 |
| Citric acid | 5.0 |
| Sodium 2-naphthalenesulfonate | 6.0 |
| 2-Benzyloxyethanol | 7.5 |
| Ethanol | 10.0 |
| Oxyquinoline sulfate | 0.04 |
| 48 wt.% aq. soln. of caustic soda | q.s.(to pH 3.0) |
| Purified water | Balance |
| | 100.0 |

Second Pack Y [2-NSA free]

2-NSA was not added in the composition of the second pack X, and the composition was balanced with purified water.

<Treatment and Ranking Methods>

The treatments of Comparative Example 1 and Examples 1-A to 1-I shown in Table 1 were applied to the natural extremely curly hair of a Japanese subject, separately. From the external appearances of the hair after drying, their correcting effects were ranked in accordance with the below-described standard.

(1) Treatment with the first packs

Each first pack was applied to the hair in an amount 1.5 times as much as the weight of the hair. The hair was smoothed into straight configuration and was left over for 30 minutes. Upon elapsed time of 15 minutes after the application, however, the hair was again smoothed into straight configuration with fingers or a comb.

(2) Intermediate treatment

In each of Examples 1-F to 1-I, the corresponding intermediate treatment was applied to the hair in an amount 5 times as much as the amount of the first pack used, and after having the intermediate treatment absorbed in the hair, the intermediate treatment was promptly rinsed off with water and the hair was towel-blotted to remove water.

In each of Examples 1-D and 1-E, the first pack was rinsed off with water, the hair was towel-blotted to remove water, the corresponding intermediate treatment was applied to the hair in an amount 1.5 times as much as the weight of the hair, the hair was smoothed into straight configuration and was left over for 30 minutes, and the hair was rinsed with water and towel-blotted to remove water.

In each of Examples 1-B and 1-C, the corresponding first pack was not rinsed off by water, and in each of Comparative Example 1 and Example 1-A, the hair was towel-blotted to remove water after the corresponding first pack was rinsed off. In all of Examples 1-A, 1B and 1-C and Comparative Example 1, no intermediate treatment was conducted.

(3) Treatment with the second packs

In each of Comparative Example 1, Example 1-A and Examples 1-D to 1-I, the corresponding second pack was applied to the hair in an amount equal in weight to the hair, the hair was smoothed into straight configuration and left over for 15 minutes, and the hair was rinsed with water and then dried. In each of Examples 1-B and 1-C, the corresponding second pack was applied to the hair in an amount 1.5 times as much as the weight of the hair, the hair was smoothed into straight configuration and left over for 15 minutes, and the hair was rinsed with water and then dried.

<Ranking standard>

5: Substantially completely straight.
4: Extremely high correcting effect compared with the comparative example.
3: Considerably high correcting effect compared with the comparative example.
2: Slightly high correcting effect compared with the comparative example.
1: Extremely natural curliness remains (comparative example).

<Results>

The results are presented in Table 1.

Table 1

| Treatment | First pack | Water rinse | Intermediate treatment | Water rinse | Second pack | Correcting effect |
|---|---|---|---|---|---|---|
| Comp. Ex. 1 | Y | Water rinse | - | - | Y | 1 |
| Ex. 1-A | Y | Water rinse | - | - | X | 3 |
| Ex. 1-B | X | - | - | - | Y | 3 |
| Ex. 1-C | X | - | - | - | X | 3 |
| Ex. 1-D | Y | Water rinse | X | Water rinse | Y | 3 |
| Ex. 1-E | Y | Water rinse | X | Water rinse | X | 3 |
| Ex. 1-F | X | - | Y | Water rinse | Y | 4 |
| Ex. 1-G | X | - | Y | Water rinse | X | 5 |
| Ex. 1-H | X | - | X | Water rinse | Y | 5 |
| Ex. 1-I | X | - | X | Water rinse | X | 5 |

As shown in Table 1, compared with Comparative Example 1, a high correcting effect was obtained in each treatment process in which 2-NSA was caused to act under an acidic condition at any one of reduction and oxidation stages. Especially when 2-NSA was added to the first pack and the intermediate treatment was conducted with one of the intermediate treatments having buffer capacity (Examples 1-F to 1-I), a superb correcting effect was obtained. When 2-NSA was added to one or both of the intermediate treatments and the second packs, a particularly superb correcting effect was obtained (Examples 1-G to 1-I).
When the hair samples which had been treated under the conditions of Examples 1-F to 1-I, respectively, were washed repeatedly seven times, some recovery of the natural curliness was observed. A composition of the same formulation as the intermediate treatment X was applied as an after-treatment to those hair samples in amounts 1.5 times as much as the weights of the hair samples. After the hair samples were left over at room temperature for 30 minutes, they were rinsed and dried. From their external appearances, correcting effects were ranked. In all of those experiments, the hair samples were confirmed to substantially return into their configurations shortly after their treatments in Examples 1-F to 1-I.

Example 2 and Comparative Example 2 (not according to the invention as claimed)

The first pack Y, intermediate treatment X and second pack Y, which were employed in Example 1, and the following intermediate treatment Z and second pack Z were employed in the below-descried extremely curly hair correcting treatments and their ranking.

<u>Intermediate Treatment Z [2-NSA added, no buffer capacity]</u>

The following intermediate treatment which was added with 2-NSA but did not have buffer capacity was prepared. This substantially corresponds to the formulation of the intermediate treatment in Example 3 of JP-A-07101840.

| | (wt.%) |
|---|---|
| Sodium 2-naphthalenesulfonate | 6.0 |
| 2-Benzyloxyethanol | 7.5 |
| Ethanol | 10.0 |
| 0.1 N hydrochloric acid | q.s.(to pH 3.0) |
| Purified water | Balance |
| | 100.0 |

Second Pack Z [aqueous sodium bromate solution]

A 6.0 wt.% aqueous solution of sodium bromate was prepared. This corresponds to the formulation of the second pack in Example 3 of JP-A-07101840.

<Treatment and Ranking Methods>

The treatments of Comparative Example 2 and Example 2 presented in Table 2 were applied to the natural extremely curly hair of a Japanese subject, separately. From the external appearances of those hair samples after drying, their correcting effects were ranked.

Table 2

| Treatment | First pack | Water rinse | Intermediate treatment | Water rinse | Second pack |
|---|---|---|---|---|---|
| Comp. Ex. 2 | Y | Water rinse | Z | Water rinse | Z |
| Ex. 2 | Y | Water rinse | X | Water rinse | Y |

(1) Treatment with the first packs

The first pack Y was applied to the hair in an amount 1.5 times as much as the weight of the hair. The hair was smoothed into straight configuration and was left over for 30 minutes. Upon elapsed time of 15 minutes after the application, however, the hair was again smoothed into straight configuration with fingers or a comb.

(2) Intermediate treatment

After the first pack was rinsed off with water and the hair was towel-blotted to remove water, the corresponding intermediate treatment was applied to the hair in an amount 1.5 times as much as the weight of the hair. The hair was smoothed into straight configuration and was left over at 50°C for 15 minutes, and the hair was rinsed with water and towel-blotted to remove water.

(3) Treatment with the second packs

The corresponding second pack was applied to the hair in an amount equal in weight to the hair, the hair was smoothed into straight configuration and left over for 15 minutes, and the hair was rinsed with water and then dried.

<Results>

Compared with the treatment by the process described in Example 3 of JP-A-07101840 (Comparative Example 2), the treatment by the process of the present invention, which makes use of an intermediate treatment having buffer capacity, (Example 2) clearly exhibited a high correcting effect.

Examples 3 and Comparative Example 3

Prepared were first packs, intermediate treatment and second packs, which had the compositions presented in Tables 3 to 5, respectively, and contained various organic acids (A). In each pack, the corresponding organic acid(s) (A) was(were) added in a total amount of 0.39 mol.

Kinds of compounds (A):

Sodium 2-naphthalenesulfonate (2-NSA)
Sodium octanesulfonate (OSA)
Sodium 2-ethylhexylsulfonate (2-EHSA)
Sodium butoxypropanesulfonate (BOPSA)
Sodium xylenesulfonate (XSA)
Sodium cumenesulfonate (CSA)

Table 3

| (wt.%) | First pack | | | | | | | | |
|---|---|---|---|---|---|---|---|---|---|
| | A* | B | C | D* | E* | F* | G | H* | Control |
| Monoethanolamine thioglycolate (50 wt.%) | 15.0 | | | | | | | | |
| Monoethanolamine | q.s. (to pH 9.15) | | | | | | | | |
| 2-Benzyloxyethanol | 7.5 | | | | | | | | |
| Ethanol | 10.0 | | | | | | | | |
| Disodium edetate | 0.5 | | | | | | | | |
| Sodium 2-naphthalenesulfonate (2-NSA) | 9.0 | - | - | - | - | - | 4.5 | 4.5 | - |
| Sodium octanesulfonate (OSA) | - | 8.5 | - | - | - | - | 4.3 | - | - |
| Sodium 2-ethylhexylsulfonate (2-EHSA) | - | - | 8.5 | - | - | - | - | - | - |
| Sodium butoxypropanesulfonate (BOPSA) | - | - | - | 8.5 | - | - | - | - | - |
| Sodium xylenesulfonate (XSA) | - | - | - | - | 8.1 | - | - | 4.1 | - |
| Sodium cumenesulfonate (CSA) | - | - | - | - | - | 8.7 | - | - | - |
| Purified water | Balance | | | | | | | | |
| Total | 100.0 | | | | | | | | |
| * not according to the invention as claimed. | | | | | | | | | |

Table 4

| (wt.%) | Intermediate treatment |
|---|---|
| Lactic acid | 5.0 |
| Citric acid | 5.0 |
| 48 wt.% aqueous solution of caustic soda | q.s. (to pH 2.9) |
| Purified water | Balance |
| Total | 100.0 |

Table 5

| (wt. %) | Second pack | | | | | | | | |
|---|---|---|---|---|---|---|---|---|---|
| | A | B | C | D | E | F | G | H | Control |
| Hydrogen peroxide (35 wt.%) | 5.71 | | | | | | | | |
| Lactic acid | 5.0 | | | | | | | | |
| Citric acid | 5.0 | | | | | | | | |

(continued)

| (wt. %) | Second pack | | | | | | | | |
|---|---|---|---|---|---|---|---|---|---|
| | A | B | C | D | E | F | G | H | Control |
| Benzyl alcohol | 7.5 | | | | | | | | |
| Ethanol | 10.0 | | | | | | | | |
| Oxyquinoline sulfate | 0.04 | | | | | | | | |
| 48 wt.% aq. soln. of caustic soda | q.s. (to pH 3.0) | | | | | | | | |
| Sodium 2-naphthalenesulfonate (NSA) | 9.0 | - | - | - | - | - | 4.5 | 4.5 | - |
| Sodium octanesulfonate (OSA) | - | 8.5 | - | - | - | - | 4.3 | - | - |
| Sodium 2-ethylhexylsulfonate (2-EHSA) | - | - | 8.5 | - | - | - | - | - | - |
| Sodium butoxypropanesulfonate (BOPSA) | - | - | - | 8.5 | - | - | - | - | - |
| Sodium xylenesulfonate (XSA) | - | - | - | - | 8.1 | - | - | 4.1 | - |
| Sodium cumenesulfonate (CSA) | - | - | - | - | - | 8.7 | - | - | - |
| Purified water | Balance | | | | | | | | |
| Total | 100.0 | | | | | | | | |

Using those first packs, intermediate treatment and second packs, correction treatments were applied to the natural extremely curly hair of a Japanese subject by the processes presented in Table 6, respectively. From the external appearances of the hair samples after drying, their correcting effects were ranked in accordance with the same standard as in Example 1.

(1) Treatment with the first packs

Each first pack was applied to the hair in an amount 2 times as much as the weight of the hair. The hair was smoothed into straight configuration and was left over for 45 minutes.

(2) Intermediate treatment

In each example, the intermediate treatment was applied to the hair in an amount 7 times as much as the used amount of the corresponding first pack without rinsing off the first pack with water. After having the intermediate treatment absorbed in the hair, the intermediate treatment was promptly rinsed off with water and the hair was towel-blotted to remove water.

In the comparative example, no intermediate treatment was conducted, the first pack was rinsed off with water, and the hair was towel-blotted to remove water.

(3) Treatment with the second packs

Each second pack was applied to the hair in an amount 1.5 times as much as the weight of the hair, the hair was smoothed into straight configuration and left over for 20 minutes, and the hair was rinsed with water and then dried.

<Results>

The results are presented in Table 6. In each of the examples except for Comparative Example 3, a high correcting effect was confirmed.

Table 6

| Treatment | First pack | Water rinse | Intermediate treatment | Water rinse | Second pack | Correcting effect |
|---|---|---|---|---|---|---|
| Comp. Ex. 3 | Control | - | - | Water rinse | Control | 1 |
| Ex. 3-A* | A* | - | Conducted | Water rinse | A | 5 |

(continued)

| Treatment | First pack | Water rinse | Intermediate treatment | Water rinse | Second pack | Correcting effect |
|---|---|---|---|---|---|---|
| Ex. 3-B | B | - | Conducted | Water rinse | B | 4 |
| Ex. 3-C | C | - | Conducted | Water rinse | C | 4 |
| Ex. 3-D* | D* | - | Conducted | Water rinse | D | 3 |
| Ex. 3-E* | E* | - | Conducted | Water rinse | E | 5 |
| Ex. 3-F* | F* | - | Conducted | Water rinse | F | 5 |
| Ex. 3-G | G | - | Conducted | Water rinse | G | 5 |
| Ex. 3-H* | H* | - | Conducted | Water rinse | H | 5 |
| * not according to the invention as claimed | | | | | | |

Example 4 and Comparative Example 4 (not according to the invention as claimed)

<Treatment and Ranking Methods>

Using the first packs X and Y, the intermediate treatment Y and the second packs X and Y prepared in Example 1, treatments were applied to the extremely natural extremely curly hair of a Latin American subject and that of a black subject in a similar manner as in Example 1-G and Comparative Example 1, separately. From the external appearances of the hair samples after drying, their natural curliness correcting effects were ranked in accordance with the same standard as in Example 1.

(1) Treatment with the first packs

Each first pack was applied in an amount 2 times as much as the weight of the Latin American subject's hair and in an amount 3 times as much as the weight of the black subject's hair. The hair samples were smoothed into straight configuration and were left over for 30 minutes. In each of these cases, however, upon an elapsed time of 15 minutes after the application, the hair sample was smoothed into straight configuration with fingers or a comb.

(2) Intermediate treatment

In each example, the intermediate treatment was applied to the hair in an amount 5 times as much as the used amount of the corresponding first pack without rinsing off the first pack with water. After having the intermediate treatment absorbed in the hair, the intermediate treatment was promptly rinsed off with water and the hair was towel-blotted to remove water.
In the comparative example, the first pack was rinsed off with water, the hair was towel-blotted to remove water, and no intermediate treatment was conducted.

(3) Treatment with the second packs

Each second pack was applied to the hair in a weight equal to the hair, the hair was smoothed into straight configuration and left over for 15 minutes, and the hair was rinsed with water and then dried.

<Results>

With respect to both of the Latin American subject's hair and the black subject's hair, practically no correcting effect was observed in the comparative example (ranking score = 1). In the examples, an extremely high correcting effect was obtained (ranking score = 4).

Example 5 (not according to the invention as claimed)

<Treatment and Ranking Methods>

Using the first pack X, the intermediate treatment Y and the second packs X and Y prepared in Example 1,

treatments were applied to the natural extremely curly hair of a Latin American subject in a similar manner as in Example 1-G and Examples 5-A to 5-D presented in Table 7, separately. From the external appearances after drying, their natural curliness correcting effects were ranked.

Table 7

| Treatment | First pack | Water rinse | Intermediate treatment | Water rinse | Ironing | Second pack | Ironing |
|---|---|---|---|---|---|---|---|
| Ex. 1-G | X | - | Y | Water rinse | - | X | - |
| Ex. 5-A | x | - | Y | Water rinse | 170°C | Y | - |
| Ex. 5-B | X | - | Y | Water rinse | 1700°C | X | - |
| Ex. 5-C | X | - | Y | Water rinse | - | Y | 170°C |
| Ex. 5-D | X | - | Y | Water rinse | - | X | 170°C |

(1) Treatment with the first pack

The first pack was applied to the hair in an amount 1.5 times as much as the weight of the hair. The hair was smoothed into straight configuration and was left over for 30 minutes. Upon an elapsed time of 15 minutes after the application, however, the hair was smoothed into straight configuration with fingers or a comb.

(2) Intermediate treatment and high-temperature ironing

The intermediate treatment was applied to the hair in an amount 5 times as much as the used amount of the first pack without rinsing off the first pack with water. After having the intermediate treatment absorbed in the hair, the intermediate treatment was promptly rinsed off with water and the hair was towel-blotted to remove water. In each of Examples 5-A and 5-B, the hair was dried with a drier and then, high-temperature ironing (170°C) was conducted by a method known per se in the art.

(3) Treatment with the second packs and high-temperature ironing

The second pack was applied to the hair in a weight equal to the hair, the hair was smoothed into straight configuration and left over for 15 minutes, and the hair was rinsed with water and then dried. In each of Examples 5-C and 5-D, high-temperature ironing (170°C) was conducted by a method known per se in the art.

Upon completion of the above treatment, the hair was fully moistened again with water and was then dried. Based on the configuration of the hair in that state, its curliness correcting effect was ranked.

<Results>

In each of the Examples 5-A to 5-D, a correcting effect superior to the result of Example 1-G was obtained.

Example 6 and Comparative Example 5

The below-described first packs X' and Y', intermediate treatment Y' and second packs X' and Y' were prepared, and were employed in the below-descried permanent wave treatments and their ranking.

<u>First Pack X' [sodium octanesulfonate (OSA) added]</u>

| | (wt.%) |
|---|---|
| Ammonium thioglycolate (50 wt.%) | 13.6 |
| Strong aqueous ammonia (28 wt.%) | q.s.(to pH 8.5) |
| Ammonium bicarbonate | 2.0 |
| Sodium octanesulfonate | 12.0 |
| Disodium edetate | 0.5 |
| Purified water | Balance |

(continued)

| | (wt.%) |
|---|---|
| | 100.0 |

First Pack Y' [OSA free] (not according to the invention as claimed)

OSA was not added in the composition of the first pack X', and the composition was balanced with purified water.

Intermediate Treatment Y' [OSA free]

| | (wt.%) |
|---|---|
| Glycolic acid | 10.0 |
| 48 wt.% aq. soln. of caustic soda | q.s.(to pH 3.0) |
| Purified water | Balance |
| | 100.0 |

Second Pack X' [OSA added]

| | (wt.%) |
|---|---|
| 35 wt.% hydrogen peroxide | 5.71 |
| Sodium octanesulfoante | 8.0 |
| Glycolic acid | 10.0 |
| Oxyquinoline sulfate | 0.04 |
| 48 wt.% aq. soln. of caustic soda | q.s.(to pH 2.6) |
| Purified water | Balance |
| | 100.0 |

Second Pack Y' [OSA free]

OSA was not added in the composition of the second pack X', and the composition was balanced with purified water.

<Treatment and Ranking Methods>

Tresses were each prepared with 10 fibers of the natural extremely curly hair of a Japanese subject. Those tresses were wound on glass rods of 10 mm in diameter, and then subjected to the treatments presented in Table 8, respectively. From their external appearances, the conditions of the thus-applied permanent waves were ranked.

Table 8

| Treatment | First pack | Water rinse | Intermediate treatment | Water rinse | Second pack |
|---|---|---|---|---|---|
| Comp. Ex. 5 | Y' | - | - | Water rinse | Y' |
| Ex. 6 | X' | - | Y' | Water rinse | X' |

(1) Treatment with the first packs

In each of Example 6 and Comparative Example 5, the corresponding first pack was applied in a weight equal to the tress, and the tress was left over at 40°C for 30 minutes.

(2) Intermediate treatment

In Example 6, the intermediate treatment was applied to the hair in an amount 2 times as much as the used amount of the corresponding first pack without rinsing off the first pack with water. After the hair was left over for 1 minute, the hair was rinsed with water and towel-blotted to remove water.

In Comparative Example 5, the first pack was rinsed off with water, and the hair was towel-blotted to remove water.

(3) Treatment with the second packs

In each of Example 6 and Comparative Example 5, the corresponding second pack was applied to the hair in a weight equal to the hair. After the hair was left over at 40°C for 20 minutes, the hair was rinsed with water, unwound from the rod, and then allowed to dry naturally.

<Results>

In Comparative Example 5, the tress was in a disheveled form accompanied with natural curliness and curls imparted by the permanent wave treatment. In Example 6, on the other hand, new curls were imparted subsequent to correction of the natural curliness. The tress was, therefore, in an attractively styled, wavy form.

**Claims**

1. A hair treatment process comprising the following steps (2-1) to (2-5) in at least one of which shaping of hair is conducted with optional use of a heating device as needed, and is conducted with a first pack comprising the organic acid (A) as defined below:

    Steps:

    (2-1) applying said first pack, which comprises a hair keratin reducing substance, to said hair, and after allowing said hair to stand for 1 to 120 minutes,
    (2-2) applying an intermediate treatment, which has buffer capacity and has a pH of from 1 to 5, to said hair,
    (2-3) rinsing said hair with water as needed,
    (2-4) applying a second pack, which comprises an oxidizing agent, to said hair, and after allowing said hair to stand for 1 to 60 minutes,
    (2-5) rinsing said hair with water, and drying said hair.

    Organic acid (A) being a compound selected from octanesulfonic acid and ethylhexanesulfonic acid or a sodium salt thereof, a potassium salt thereof or an ammonium salt thereof.

2. The hair treatment process according to claim 1, wherein said intermediate treatment also comprises said organic acid (A).

3. A hair treatment process according to claim 1, wherein said second pack also comprises said organic acid (A), has buffer capacity, and has a pH of from 1 to 5.

4. A hair treatment process comprising applying an aftertreatment, which comprises said organic acid (A), has buffer capacity and has a pH of from 1 to 5, to said hair treated by the process described in any one of claims 1-3, allowing said hair to stand for 1 to 120 minutes, rinsing said hair with water, and then drying said hair.

**Patentansprüche**

1. Haarbehandlungsverfahren, umfassend die folgenden Schritte (2-1) bis (2-5), worin bei zumindest einem die Formgebung des Haars durch wahlweise Verwendung einer Erwärmungsvorrichtung nach Bedarf durchgeführt wird, und das mit einer ersten Packung durchgeführt wird, umfassend die organische Säure (A) wie unten definiert:

    Schritte:

(2-1) Auftragen der ersten Packung, die eine Haarkeratin reduzierende Substanz enthält, auf das Haar, und nach Stehenlassen des Haares für 1 bis 120 Minuten, (2-2) Auftragung einer Zwischenbehandlung, die eine Pufferkapazität aufweist und einen pH von 1 bis 5 hat, auf das Haar,
(2-3) Spülen des Haars mit Wasser nach Bedarf,
(2-4) Auftragen einer zweiten Packung, die ein Oxidationsmittel enthält, auf das Haar und nach Stehenlassen des Haars für 1 bis 60 Minuten,
(2-5) Spülen des Haars mit Wasser und Trocknen des Haars,

worin die organische Säure (A) eine Verbindung ist, ausgewählt aus Octansulfonsäure und Ethylhexansulfonsäure oder einem Natriumsalz davon, Kaliumsalz davon oder Ammoniumsalz davon.

**2.** Haarbehandlungsverfahren nach Anspruch 1, worin die Zwischenbehandlung ebenfalls die organische Säure (A) enthält.

**3.** Haarbehandlungsverfahren nach Anspruch 1, worin die zweite Packung ebenfalls die organische Säure (A) enthält, eine Pufferkapazität hat und einen pH von 1 bis 5 hat.

**4.** Haarbehandlungsverfahren, umfassend das Auftragen einer Nachbehandlung, umfassend die organische Säure (A), die eine Pufferkapazität hat und einen pH von 1 bis 5 hat, auf das Haar, behandelt durch das Verfahren nach einem der Ansprüche 1 bis 3, Stehenlassen des Haars für 1 bis 120 Minuten, Spülen des Haars mit Wasser und anschließendes Trocknen des Haars.

## Revendications

**1.** Un procédé de traitement des cheveux comprenant les étapes suivantes (2-1) à (2-5) dans au moins l'une desquelles la mise en forme des cheveux est effectuée avec l'utilisation optionnelle d'un dispositif de chauffage selon le besoin, et est effectué avec un premier paquet comprenant l'acide organique (A) tel que défini ci-dessous:

Étapes:

(2-1) application dudit premier paquet, qui comprend une substance réduisant la kératine des cheveux, auxdits cheveux, et après laisser lesdits cheveux reposer pendant 1 à 120 minutes,
(2-2) application d'un traitement intermédiaire, qui a une capacité tampon et a un pH de 1 à 5, auxdits cheveux, (2-3) rinçage desdits cheveux avec de l'eau selon le besoin,
(2-4) application d'un second paquet, qui comprend un agent oxydant, auxdits cheveux, et après laisser lesdits cheveux reposer pendant 1 à 60 minutes,
(2-5) rinçage desdits cheveux avec de l'eau, et séchage desdits cheveux.

L'acide organique (A) étant un composé choisi parmi l'acide octanesulfonique et l'acide éthylhexanesulfonique ou un sel de sodium de ceux-ci, un sel de potassium de ceux-ci ou un sel d'ammonium de ceux-ci.

**2.** Le procédé de traitement des cheveux selon la revendication 1, dans lequel ledit traitement intermédiaire comprend également ledit acide organique (A).

**3.** Un procédé de traitement des cheveux selon la revendication 1, dans lequel ledit second paquet comprend également ledit acide organique (A), a une capacité tampon, et a un pH de 1 à 5.

**4.** Un procédé de traitement des cheveux comprenant l'application d'un après-traitement, qui comprend ledit acide organique (A), a une capacité tampon et a un pH de 1 à 5, auxdits cheveux traités par le procédé décrit dans l'une quelconque des revendications 1-3, le repos desdits cheveux pendant 1 à 120 minutes, le rinçage desdits cheveux avec de l'eau, et alors le séchage desdits cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 08092043 A
- JP 07101840 A